# EUROPÄISCHE PATENTSCHRIFT

(11) **EP 3 198 274 B1**
(45) Veröffentlichungstag und Bekanntmachung des Hinweises auf die Patenterteilung: **06.11.2019**
(21) Anmeldenummer: 15794816.7
(22) Anmeldetag: 02.09.2015
(51) Int. Cl.: G01N 33/543, G01N 33/569, G01N 33/58

(54) **ANTIGENSPEZIFISCHE IMMUNFÄRBUNG VON T-ZELLEN**
ANTIGEN-SPECIFIC IMMUNOSTAINING OF T CELLS
COLORATION IMMUNOLOGIQUE DE LYMPHOCYTES T SPÉCIFIQUE À UN ANTIGÈNE

(30) Priorität: 22.09.2014 DE 102014113688
(43) Veröffentlichungstag der Anmeldung: 02.08.2017
(73) Patentinhaber: Jacobs University Bremen gGmbH, 28759 Bremen (DE)
(72) Erfinder: SPRINGER, Sebastian Hartmut, 28717 Bremen (DE)
(74) Vertreter: Weidner Stern Jeschke
(86) Internationale Anmeldenummer: PCT/DE2015/100365
(87) Internationale Veröffentlichungsnummer: WO 2016/045660

(56) Entgegenhaltungen:
- WO-A1-2009/003492
- WO-A2-2010/037395
- AU-A1- 2004 203 482
- DE-A1- 10 010 264
- DE-A1- 10 042 023
- Anonymous: "Polyelectrolyte - Wikipedia, the free encyclopedia", , 5. Januar 2016 (2016-01-05), Seiten 1-4, XP055238793, Gefunden im Internet: URL:https://en.wikipedia.org/w/index.php?t itle=Special:Book&bookcmd=download&collect ion_id=c1ad7e07807c26b76240127648c7840ee50 362af&writer=rdf2latex&return_to=Polyelect rolyte [gefunden am 2016-01-05]

## Beschreibung

Die Erfindung betrifft ein Verfahren zur antigenspezifischen Immunfärbung von T-Zellen sowie ein Färbekit hierfür.

MHC-Klasse-I-Proteine sind an der zellulären Immunantwort beteiligte Transmembranproteine, die antigene Peptide aus dem Zellinneren einer antigenpräsentierenden Zelle (APC), beispielsweise aus dem Cytosol oder dem Lumen der endocytischen Organellen, binden und an der Zelloberfläche den zytotoxischen T-Zellen (CTL) präsentieren (Klasse-I-Antigenpräsentation). Die Bindung eines T-Zell-Rezeptors (TCR) einer CTL an einen Klasse-I-Peptid-Komplex einer APC führt, abhängig vom Ort der Reaktion im Körper, dem Typ der APC (B-Zelle, dendritische Zelle, etc.) und dem Aktivierungszustand der CTL, zur Aktivierung der CTL und/oder zur Induktion des Zelltods (Apoptose) der APC durch die CTL. Die Spezifität (Reaktionsfähigkeit mit einem bestimmten Klasse-I-Peptid-Komplex) einer CTL liegt dabei darin begründet, dass sie an ihrer Oberfläche nur eine Art TCR (d.h. nur TCRs einer einzelnen eindeutigen Sequenz) trägt. Die Immunantwort ist effektiv, weil solche CTL, die mit Selbst-Peptiden (Peptiden, die die aus körpereigenen Proteinen erzeugt werden) reagieren, im Thymus eliminiert werden. Aus diesem Grund bedeutet die Erkennung eines antigenen Peptids durch den TCR einer CTL, dass die APC fremde Proteine produziert, die von Viren oder intrazellulären Parasiten (Bakterien, Protozoen) stammen könnten. Auch eine Überproduktion endogener Peptide in malignen entarteten Zellen, zum Beispiel in Tumoren, kann zu solchen Erkennungsreaktionen führen.

Nahezu alle in der Zelle vorhandenen Proteine werden in Peptide zersetzt, die dann im endoplasmatischen Retikulum (ER), einer membranumgrenzten Organelle im Zellinneren, an MHC-Klasse-I-Proteine binden. Anschließend wird der Komplex aus Peptid und MHC-Klasse-I-Protein (pMHC-I) an die Zelloberfläche transportiert und steht dort für die Erkennung durch CTL zur Verfügung. Wenn nun aufgrund einer tumorigenen malignen Entartung neuartige oder mutierte Proteine produziert werden, oder wenn durch eine virale oder bakterielle Infektion virale oder bakterielle Proteine aus dem genetischen Material des Virus oder Bakteriums produziert werden, werden diese ,neuartigen' Proteine ebenfalls in Peptide zersetzt, die dann im Komplex mit MHC-Klasse-I-Proteinen auf der Zelloberfläche präsentiert werden. Diese ,neuartigen' Peptide sind verschieden von den zelleigenen und lösen eine Erkennung durch die CTL aus. Die Präsentation durch MHC-Klasse-I-Proteine spielt ebenfalls eine Rolle in allergischen Reaktionen, der Abstoßung von Transplantaten und einer Anzahl von Autoimmunkrankheiten wie multipler Sklerose und Spondyloarthropathien (z.B. Morbus Bechterew oder rheumatoider Arthritis).

Die Untersuchung der Immunantworten, die durch MHC-Klasse-I-Proteine vermittelt werden, erfordert oft den Nachweis derjenigen CTL, die mit einem bestimmten Klasse-I-Peptid-Komplex (Epitop) reagieren. Reaktionen auf ein einziges immunodominantes Epitop machen oft 10-20% der gesamten T-Zell-Population in einem Organismus aus, und die Verfolgung der T-Zell-Frequenz (d.h. des Anteils derjenigen CTL, die mit einem bestimmten Epitop reagieren, an der Gesamtmenge der CTL im Organismus) erlaubt es beispielsweise, die Immunantwort (und z.B. Gelingen oder Fehlschlagen einer Therapie) genau zu verfolgen. Entsprechende Nachweisverfahren sind beispielsweise in Hadrup und Schumacher 2010 (Hadrup SR, Schumacher TN 2010. MHC-based detection of antigen-specific CD8+ T cell responses. Cancer Immunol Immunother.59(9):1425-33. doi: 10.1007/s00262-010-0824-2) und Andersen et al. 2012 (Andersen RS, Kvistborg P, Frøsig TM, Pedersen NW, Lyngaa R, Bakker AH, Shu CJ, Straten Pt, Schumacher TN, Hadrup SR. 2012. Parallel detection of antigen-specific T cell responses by combinatorial encoding of MHC multimers. Nat Protoc. 7(5):891-902. doi: 10.1038/nprot.2012.037) beschrieben.

Anikeeva et al 2006 (Anikeeva N, Lebedeva T, Clapp AR, Goldman ER, Dustin ML, Mattoussi H, Sykulev Y. 2006, Quantum dot/peptide-MHC biosensors reveal strong CD8-dependent cooperation between self and viral antigens that augment the T cell response, Proc Natl Acad Sci U.S.A. 103(45):16846-51) beschreiben ein Verfahren, bei dem lumineszierende Quantenpunkte ("Quantum Dots"; QD) aus Halbleitermaterial zur Untersuchung der Wechselwirkung zwischen MHC-Klasse-I-Proteinen und CTL eingesetzt werden. Bei dem Verfahren werden zur Imitierung von APC fluoreszenzmarkierte mit viralen Antigenen beladene MHC-Klasse-I-Proteine (pMHC-I-Proteine) mit ihrem C-Terminus auf einem solchen Quantenpunkt immobilisiert und mit antiviralen CD8⁺-T-Zellen in Kontakt gebracht.

WO 2010/037395 A2 und WO 2009/003492 A1 beschreiben MHC-Multimere bestehend aus einer Multimerisierungsdomäne, an die ein oder mehrere MHC-Peptid-Komplexe durch einen oder mehrere Linker gebunden sind. Bei der Multimerisierungsdomäne kann es sich um Kügelchen, zellähnliche Strukturen, einschließlich membranbasierter Strukturen, beispielsweise Micellen, Liposomen und andere Membranstrukturen handeln. Das MHC-Multimer kann zur Identifizierung antigenspezifischer T-Zellen mittels Durchflusszytometrie verwendet werden, wobei das MHC-Multimer mit einem Fluoreszenzfarbstoff markiert ist.

AU 2004203482 A1 beschreibt Membranvesikel (Exosomen), die rekombinante MHC-Moleküle oder Peptid-MHC-Komplexe enthalten oder tragen, und deren Verwendung als immunogene Mittel oder diagnostische oder therapeutische Werkzeuge. Die Vesikel können in ihrem Inneren oder an ihrer Oberfläche einen Marker, beispielsweise Fluoreszenzmarker, aufweisen.

DE 10042023 A1 beschreibt Polyelektrolyt-Kapseln, bei denen feste Teilchen signalerzeugender Substanzen, z.B. Fluorophore, von einer oder mehreren Schichten eines Polyelektrolyten eingekapselt sind, und die an ihrer äußeren Oberfläche Affinitätsmoleküle tragen, die Zielmoleküle in einer Probe spezifisch erkennen und daran binden. Die Polyelektrolyt-Kapseln finden Verwendung zur Signalerzeugung beim optischen, elektrochemischen oder chemischen Nachweis von Zielmolekülen.

WO2010037395 beschreibt MHC-Peptidkomplexe und deren Verwendung zur Diagnose, Behandlung oder Impfung gegen eine Krankheit bei einer Person. Genauer gesagt, offenbart die Erfindung MHC-Komplexe, die krebsantigene Peptide umfassen, und deren Verwendung.

WO2009003492 beschreibt Verfahren zur Herstellung, Kennzeichnung und Verwendung von MHC-Multimeren, insbesondere neue Methoden zur Erzeugung von MHC-Multimeren und Methoden zur Verbesserung bestehender und neuer MHC-Multimere. Die Erfindung beschreibt auch verbesserte Methoden für den Einsatz von MHC-Multimeren bei der Analyse von T-Zellen einschließlich diagnostischer und prognostischer Methoden.

AU2004203482 betrifft Membranvesikel, die Moleküle, insbesondere antigene Moleküle, mit vorgegebener Struktur umfassen, und ihre Verwendung, insbesondere Vesikel (Exosomen), die rekombinante Moleküle des großen Histokompatibilitätskomplexes umfassen, und ihre Verwendung als Immunogen oder für diagnostische und therapeutische Zwecke. Die Erfindung betrifft auch Verfahren zur Herstellung der Vesikel, genetische Konstrukte, Zellen und Zusammensetzungen, die für die Durchführung der Verfahren nützlich sind.

DE 10042023 betrifft betrifft Kapseln, die feste Teilchen signalerzeugender Substanzen einkapseln und an ihrer äußeren Oberfläche Affinitätsmoleküle tragen, die Zielmoleküle in einer Probe spezifisch erkennen und daran binden. Gegenstand der Erfindung ist die Verwendung dieser Kapseln zur Signalerzeugung beim optischen, elektrochemischen oder chemischen Nachweis von Zielmolekülen. Es wird ein Nachweisverfahren und ein Kit beschrieben.

Aufgabe der vorliegenden Erfindung ist es, einen einfachen und zuverlässigen antigenspezifischen Nachweis von T-Zellen zu ermöglichen.

Zur Lösung der Aufgabe stellt die vorliegende Erfindung in einem ersten Aspekt ein Verfahren zur antigenspezifischen Immunfärbung von T-Zellen bereit, wobei eine Polyelektrolyt-Mikrokapsel, die außen ein antigenpräsentierendes MHC-Klasse-I-Molekül aufweist, mit einer einen T-Zell-Rezeptor tragenden T-Zelle unter Bedingungen in Kontakt gebracht wird, die eine Wechselwirkung zwischen dem T-Zell-Rezeptor und dem antigenpräsentierenden MHC-Klasse-I-Molekül erlauben, so dass ein Komplex aus T-Zelle, antigenpräsentierendem MHC-Klasse-I-Molekül und Polyelektrolyt-Mikrokapsel gebildet werden kann, so wie in Anspruch 1 definiert.

Das erfindungsgemäße Verfahren stellt einen einfach durchzuführenden, dabei aber empfindlichen antigenspezifischen Nachweis von T-Zellen oder T-Zellpopulationen bereit. Das erfindungsgemäße Verfahren ist sehr flexibel ausgestaltbar, und stellt einen einfachen und empfindlichen Nachweis mittels Fluoreszenzmarkierung einzelner oder auch mehrerer Komponenten des Komplexes aus T-Zelle, antigenpräsentierendem MHC-Klasse-I-Molekül und Polyelektrolyt-Mikrokapsel bereit.

Unter einer "Polyelektrolyt-Mikrokapsel" (abgekürzt auch PEMC) wird hier ein Hohlkörper mit einer mehrschichtigen Polyelektrolythülle verstanden, die einen mittleren Durchmesser von 100 nm bis 10 µm aufweist. Polyelektrolyt-Mikrokapseln können beispielsweise durch aufeinander folgende Adsorption entgegengesetzt geladener Polyelektrolytschichten auf einen geladenen Matrizenkörper, der anschließend aufgelöst werden kann, hergestellt werden (s. z.B. WO 1999/047252 A2; DE 10042023 C2; WO 2005057163 A2; Tong und Gao 2008, Multilayer microcapsules with tailored structures for bio-related applications, J. Mater. Chem., 2008, 18, 3799-3812).

Unter "Polyelektrolyten" werden Polymere mit ionisierbaren oder ionisch dissoziierbaren Gruppen verstanden, die Bestandteil oder Substituent der Polymerkette sein können. Dabei ist die Anzahl der ionisierbaren oder ionisch dissoziierbaren Gruppen vorzugsweise so groß, dass die Polymere in ionischer Form (Polyion) wasserlöslich oder in Wasser quellbar sind. Polyelektrolyte können linear oder verzweigt sein. Je nach Art der dissoziierbaren Gruppen unterscheidet man kationische (Polybasen) und anionische Polyelektrolyte (Polysäuren). Kationische Polyelektrolyte enthalten überwiegend positiv geladene (basische) Gruppen, anionische Polyelektrolyte überwiegend anionische (saure) Gruppen. Ein Beispiel für einen kationischen Polyelektrolyten ist Polyallylamin oder Polyallylamin-Hydrochlorid (PAH), ein Beispiel für einen anionischen Polyelektrolyten ist Polyacrylsäure (PAA).

Unter einem "MHC-Klasse-I-Molekül", "Klasse-I-Molekül", "MHC-I-Molekül", MHC-Klasse-I-Protein", "Klasse-I-Protein" oder "MHC-I-Protein" wird ein Haupthistokompatibilitätskomplex-Klasse-I-Protein verstanden. MHC-Klasse-I-Proteine sind vom Haupthistokompatibilitätskomplex (engl. "Major Histocompatibility Complex", MHC) kodierte Transmembranproteine und an der zellulären Immunantwort beteiligt. MHC-Klasse-I-Proteine binden Peptide aus dem Zellinneren, wie beispielsweise aus dem Cytosol oder dem Lumen der endocytischen Organellen und präsentieren diese an der Zelloberfläche den zytotoxischen T-Zellen als (Antigenpräsentation). Ein MHC-I-Molekül besteht in der Regel aus einer schweren α-Kette (auch kurz "hc", Molekulargewicht ca. 44 kDa, etwa 350 Aminosäuren) mit drei extrazellulären Domänen (α1, α2 und α3) und einer Transmembrandomäne sowie einer nicht kovalent daran gebundenen leichten β-Kette (auch "β2-Mikroglobulin" oder kurz "β2m", ca. 12 kDa, 99 Aminosäuren). Menschliche MHC-Klasse-I-Proteine werden auch als HLA-Proteine (HLA = human leukocyte antigen) bezeichnet. Der Begriff umfasst sowohl klassische MHC-Klasse-I-Proteine wie HLA-A, HLA-B und HLA-C als auch nicht-klassische MHC-Klasse-I-Proteine wie HLA-E, HLA-F und HLA-G. Zudem umfasst der Begriff auch peptidbindende Fragmente aller dieser Proteine, insbesondere die extrazelluläre Domäne, sowie Fusionsproteine, die aus der schweren Kette (oder einem peptidbindenden Fragment der schweren Kette), ggfs. einem Linker, und der leichten Kette bestehen. Unter einem "Fragment" werden hier insbesondere zusammenhängende Teilsequenzen von mindestens 10, 15, 20, 25, 30, 35, 40, 45, 50, 60, 65, 70, 80, 90 oder mindestens 100 Aminosäuren, bevorzugt mindestens 110, 120, 130, 140, 150, 160, 170, 180, 190 oder mindestens 200 Aminosäuren verstanden. Der Begriff "MHC-Klasse-I-Protein" umfasst auch MHC-Klasse-I-Proteine nicht-menschlicher Wirbeltierspezies, beispielsweise von Maus (Mus musculus), Ratte (Rattus norvegicus), Rind (Bos taurus), Pferd (Equus equus) oder Grüner Meerkatze (Macaca mulatta). Die MHC-Klasse-I-Proteine der Maus werden beispielsweise als H-2-Proteine bezeichnet, die von den Genloci H-2K, H-2L und H-2D kodiert werden. Ein bestimmter Allotyp eines Maus-MHC-Klasse-I-Proteins ist beispielsweise H-2Kb oder H-2Ld.

Ein Komplex aus Peptid und MHC-Klasse-I-Protein, d.h. ein MHC-Klasse-I-Protein mit daran gebundenem Peptid, wird hier auch als "Peptid/MHC-Klasse-I-Protein-Komplex", "Klasse-I-Peptid-Komplex" oder "Peptid/MHC-I" bezeichnet. Als Abkürzung für einen solchen Komplex wird auch "pMHC-I" verwendet. Der Begriff "antigenpräsentierendes MHC-Klasse-I-Protein" wird synonym zu dem Komplex verwendet. "Antigenpräsentierend" bedeutet in diesem Zusammenhang, dass das MHC-Klasse-I-Protein ein Peptid (Antigen) gebunden aufweist.

Wenn hier von einem Komplex aus Polyelektrolyt-Mikrokapsel (PEMC) und Peptid/MHC-Klasse-I-Protein (pMHC-I/PEMC-Komplex) gesprochen wird, umfasst dieser Begriff auch ein etwaiges Kopplungssystem, mit dessen Hilfe das antigenpräsentierende MHC-Klasse-I-Protein an die Polyelektrolyt-Mikrokapsel gebunden ist.

Wenn hier der Begriff "Untersuchungspeptid" verwendet wird, ist damit ein (antigenisches) Peptid gemeint, das an ein MHC-Klasse-I-Protein gebunden wird oder ist und dem erfindungsgemäßen Verfahren unterworfen wird.

Unter dem Begriff "T-Zellen" werden T-Lymphozyten verstanden. T-Lymphozyten tragen MHC-Rezeptoren (T-Zell-Rezeptoren, TCR) in ihrer Zellmembran, mit deren Hilfe sie an MHC-I-Proteine anderer Zellen gebundene Peptidantigene erkennen können. Mit dem Begriff "zytotoxische T-Zellen" (CTL) werden CD8⁺-T-Lymphozyten bezeichnet.

Geeignete Polyelektrolyt-Mikrokapseln sind dem Fachmann bekannt oder können von ihm anhand seines Fachwissens hergestellt werden. Es ist bevorzugt, Polyelektrolyt-Mikrokapseln zu verwenden, die durchschnittlich kleiner sind als durchschnittliche T-Zellen, bevorzugt um mindestens den Faktor 2, 3, 4, 5, 6, 7, 8, 9, 10, 15, 20, 30, 40 oder 50. Bevorzugte Polyelektrolyt-Mikrokapseln haben beispielsweise einen durchschnittlichen Durchmesser von 100-6000 nm, 100-900 nm, 100-800 nm, 100-700 nm, 100-600 nm, 100-500 nm, 150-300 nm, oder 3000-6000 nm.

Bereichsangaben wie "100-1000" sind hier immer so zu verstehen, dass jeder Zwischenwert mit offenbart ist. Auch ein beliebiger kleinerer Bereich aus dem Bereich soll dabei mit offenbart sein, wobei unter dem kleineren Bereich auch Bereiche zu verstehen sind, die keinen der Grenzwerte des Bereichs umfassen. So umfasst eine Angabe wie "100-1000" nicht nur Bereiche wie "100-950" oder "600-1000", sondern auch Bereiche von "250-700" oder "200-900" oder "200-400", wobei die einzelnen Werte innerhalb des Bereichs ausdrücklich mit erfasst sind, und nicht nur dessen Grenzwerte.

Der Ausdruck, wonach die Polyelektrolyt-Mikrokapsel "außen ein antigenpräsentierendes MHC-Klasse-I-Molekül aufweist", bedeutet, dass das MHC-Klasse-I-Molekül so an die Hülle der Polyelektrolyt-Mikrokapsel geheftet ist, dass das MHC-Klasse-I-Molekül mit dem das (antigenische) Peptid bindenden Bereich zur außerhalb der Polyelektrolyt-Mikrokapsel liegenden Umgebung gerichtet ist. Vorzugsweise ist das MHC-Klasse-I-Molekül mit dem C-Terminus seiner schweren Kette an die äußere Hülle der Polyelektrolyt-Mikrokapsel gebunden oder in der äußeren Hülle verankert. "Geheftet" bedeutet hier, dass unter Bedingungen, wie sie normalerweise während der Durchführung des erfindungsgemäßen Verfahrens herrschen, der Kontakt zwischen MHC-Klasse-I-Molekül und Polyelektrolyt-Mikrokapsel-Hülle nicht verloren geht. Der Begriff "geheftet" wird hier gegebenenfalls auch synonym zu den Begriffen "gebunden" oder "verankert" verwendet. Bindungsarten sind dem Fachmann bekannt und schließen beliebige kovalente und nicht-kovalente Bindungen ein.

Bei dem erfindungsgemäßen Verfahren wird ein Komplex aus antigenpräsentierendem MHC-Klasse-I-Molekül und Polyelektrolyt-Mikrokapsel (pMHC-I/PEMC-Komplex) mit einer TCR-tragenden T-Zelle so in Kontakt gebracht, dass der TCR der T-Zelle an die pMHC-I-Moleküle in dem pMHC-I/PEMC-Komplex bindet, wodurch ein Komplex aus T-Zelle, antigenpräsentierendem MHC-Klasse-I-Molekül und Polyelektrolyt-Mikrokapsel (pMHC-I/PEMC/T-Zell-Komplex) gebildet wird.

Das antigenpräsentierende MHC-Klasse-I-Molekül ist direkt oder indirekt mittels kovalenter und/oder nicht-kovalenter Bindung an die Polyelektrolyt-Mikrokapsel gebunden. Bei der direkten Bindung kann es sich beispielsweise um eine kovalente oder elektrostatische Bindung handeln. Bevorzugt ist das MHC-Klasse-I-Molekül indirekt über ein geeignetes Kopplungssystem an die Polyelektrolyt-Mikrokapsel gebunden. Unter einem "Kopplungssystem" wird ein System aus sich gegenseitig bindenden Kopplungselementen, z.B. Molekülen, verstanden, die eine stabile indirekte Verbindung zwischen der Polyelektrolyt-Mikrokapsel und dem MHC-Klasse-I-Molekül vermitteln. Geeignete Kopplungssysteme sind dem Fachmann bekannt und umfassen bevorzugt ein Kopplungssystem, bei dem ein erstes Kopplungselement mehrere entsprechend daran bindende zweite Kopplungselemente binden kann, wie beispielsweise das Avidin-Biotin und Streptavidin-Biotin-System, um die Avidität des Kopplungssystems zu erhöhen.

In einer bevorzugten Ausführungsform des erfindungsgemäßen Verfahrens ist das Kopplungssystem ein Avidin-Biotin- und/oder Streptavidin-Biotin-System, wobei Avidin oder Streptavidin, bevorzugt Steptavidin, vorzugsweise direkt an die Polyelektrolyt-Mikrokapsel und Biotin direkt an das antigenpräsentierende MHC-Klasse-I-Molekül, vorzugsweise an dessen C-Terminus, gebunden ist. "Direkt gebunden" bedeutet insbesondere kovalent gebunden. Streptavidin kann beispielsweise kovalent an freie Carboxylgruppen des Polyelektrolyten gebunden werden. Verfahren zur Biotinylierung von MHC-Klasse-I-Molekülen sind dem Fachmann gut bekannt und umfassen beispielsweise enzymatische Verfahren unter Verwendung einer Biotinligase (BirA). Aufgrund der niedrigen Affinität eines einzelnen Klasse-I-Peptid-Komplexes zu einem einzelnen TCR ist es für die Bindung einer T-Zelle an den Komplex aus Polyelektrolyt-Mikrokapsel und antigenpräsentierendem MHC-Klasse-I-Molekül (pMHC-I-Molekül) vorteilhaft, Multimere von pMHC-I-Molekül zu verwenden, die aufgrund des Aviditätseffektes fest genug an den TCR einer T-Zelle binden. Ein derartiges Multimer kann durch die Streptavidin-vermittelte Tetramerisierung von biotinylierten pMHC-I-Molekülen erhalten werden.

Bei dem erfindungsgemäßen Verfahren ist eine Fluoreszenzmarkierung des pMHC-I/PEMC/T-Zell-Komplexes vorgesehen, um beispielsweise eine Detektion mittels fluoreszenzbasierter Durchflusszytometrie (fluorescence-activated cell sorting, FACS) zu ermöglichen. Dabei wird die Fluoreszenz des Komplexes gemessen, wodurch Rückschlüsse auf Menge bzw. Anteil der mit dem pMHC-I-Molekül reagierenden T-Zellen in der untersuchten Probe gezogen werden können.

Die Fluoreszenzmarkierung kann je nach Einsatzzweck an unterschiedlichen Komponenten der genannten Komplexe vorgesehen bzw. während des Verfahrens erzeugt werden. Geeignete Fluorophore (Fluoreszenzfarbstoffe) sowie Verfahren zur Fluoreszenzmarkierung sind dem Fachmann bekannt oder leicht anhand von Routineversuchen zu ermitteln. Beispiele für geeignete Fluoreszenzfarbstoffe sind Fluorescein, Tetramethylrhodamin, die so genannten "Alexa-Fluor"-Farbstoffe (Molecular Probes Inc., Eugene, USA, z.B. Alexa Fluor® 488), die "Chromeo™"-Farbstoffe (Active Motif Inc., Carlsbad, USA, z.B. Chromeo 488), oder "DyLight®"-Farbstoffe (Dyomics GmbH, Jena, Deutschland; Thermo Fisher Scientific Inc., Rockford, USA, z.B. DyLight 488). Die Liste dieser Beispiele ist nicht erschöpfend.

Bei dem erfindungsgemäßen Verfahren wird eine Polyelektrolyt-Mikrokapsel verwendet, die innen einen Fluoreszenzfarbstoff aufweist. Bevorzugt ist der Fluroreszenzfarbstoff von der Hülle im Inneren der Polyelektrolyt-Mikrokapsel eingeschlossen. Bei dem Fluoreszenzfarbstoff im Innern der Polyelektrolyt-Mikrokapsel kann es sich um einen an Dextran oder ein Protein gekoppelten Fluoreszenzfarbstoff handeln. Der Fluoreszenzfarbstoff ist vorzugsweise nicht an einen Quantenpunkt gebunden. Der Fluoreszenzfarbstoff ist vorzugsweise so ausgewählt, dass er im Innern der Polyelektrolyt-Mikrokapsel zumindest über den Nachweiszeitraum von der Hülle der Polyelektrolyt-Mikrokapsel eingeschlossen bleibt und im Wesentlichen nicht freigesetzt wird. Durch Fluoreszenzmarkierung der Polyelektrolyt-Mikrokapsel, insbesondere bei einer Ausgestaltung mit Fluoreszenzfarbstoff(en) im Innern der Polyelektrolyt-Mikrokapsel, ist eine starke Beladung mit Fluorophoren (Fluoreszenzfarbstoffen) möglich, wodurch ein stärkeres und einfacher zu detektierendes Signal resultiert.

Im beanspruchten Verfahren weist die Polyelektrolyt-Mikrokapsel, außen ein antigenpräsentierendes MHC-Klasse-I-Molekül und innen einen ersten Fluoreszenzfarbstoff auf. Im Folgenden beschriebene Ausführungsformen, bei denen sich der Fluoreszenzfarbstoff nicht innen in der Polyelektrolyt-Mikrokapsel befindet, stellen lediglich Referenzbeispiele dar.

Alternativ oder zusätzlich kann auch ein fluoreszenzmarkiertes Kopplungssystem verwendet werden, wobei vorzugsweise ein außen an die Polyelektrolyt-Mikrokapsel gebundenes erstes Kopplungselement des Kopplungssystems, beispielsweise Avidin und/oder Streptavidin, fluoreszenzmarkiert ist. Das fluoreszenzmarkierte Kopplungselement kann beispielsweise auch durch direkte oder indirekte Bindung eines gegen das Kopplungselement gerichteten fluoreszenzmarkierten Antikörpers fluoreszenzmarkiert sein. "Indirekt" bedeutet in diesem Zusammenhang beispielsweise, dass ein primärer nicht markierter Antikörper an das Kopplungselement gebunden und ein gegen den primären Antikörper gerichteter sekundärer fluoreszenzmarkierter Antikörper an den primären Antikörper gebunden wird.

Weiter alternativ oder gegebenenfalls auch zusätzlich zu den obigen Fluoreszenzmarkierungen kann auch ein fluoreszenzmarkierter Antikörper eingesetzt werden, der direkt oder indirekt an die Polyelektrolyt-Mikrokapsel und/oder das antigenpräsentierende MHC-Klasse-I-Molekül in dem Komplex aus T-Zelle, antigenpräsentierendem MHC-Klasse-I-Molekül und Polyelektrolyt-Mikrokapsel gebunden wird. "Indirekt" bedeutet in diesem Zusammenhang beispielsweise, dass ein primärer nicht markierter Antikörper an die Polyelektrolyt-Mikrokapsel, und/oder das antigenpräsentierende MHC-Klasse-I-Molekül gebunden und ein gegen den primären Antikörper gerichteter sekundärer fluoreszenzmarkierter Antikörper an den primären Antikörper gebunden wird. Ein an die Polyelektrolyt-Mikrokapsel und/oder das antigenpräsentierende MHC-Klasse-I-Molekül bindender fluoreszenzmarkierter Antikörper wird beispielsweise bevorzugt dann eingesetzt, wenn die Polyelektrolyt-Mikrokapsel keinen Fluoreszenzfarbstoff enthält.

Bei dem erfindungsgemäßen Verfahren wird zusätzlich auch ein fluoreszenzmarkierter Antikörper eingesetzt, der direkt oder indirekt an die T-Zelle gebunden wird. "Indirekt" bedeutet in diesem Zusammenhang beispielsweise, dass ein primärer nicht markierter Antikörper an die T-Zelle gebunden und ein gegen den primären Antikörper gerichteter sekundärer fluoreszenzmarkierter Antikörper an den primären Antikörper gebunden wird. Beispielsweise kann ein fluoreszenzmarkierter Antikörper verwendet werden, der spezifisch an den T-Zell-Rezeptor oder einen Corezeptor des T-Zell-Rezeptors bindet, oder ein fluoreszenzmarkierter Antikörper, der spezifisch an einen zweiten Antikörper bindet, der spezifisch an den T-Zell-Rezeptor oder Corezeptor des T-Zell-Rezeptors bindet. Beispielsweise kann ein primärer gegen den CD8-Corezeptor gerichteter Antikörper und ein fluoreszenzmarkierter gegen den primären Antikörper gerichteter sekundärer Antikörper verwendet werden. Geeignete Antikörper sind dem Fachmann bekannt und kommerziell erhältlich.

In einer besonders bevorzugten Ausführungsform umfasst das erfindungsgemäße Verfahren folgende Schritte:
a. Herstellen einer Polyelektrolyt-Mikrokapsel, die innen und/oder außen, vorzugsweise innen, einen Fluoreszenzfarbstoff und außen ein gebundenes erstes Kopplungselement eines Kopplungssystems, vorzugsweise Avidin oder Streptavidin, aufweist,
b. Herstellen eines gefalteten, ein Antigen präsentierenden MHC-Klasse-I-Moleküls, das ein zweites Kopplungselement des Kopplungssystems, vorzugsweise Biotin, aufweist, das mit dem ersten Kopplungselement eine Bindung eingehen kann,
c. Inkontaktbringen des in Schritt a) hergestellten Komplexes aus Polyelektrolyt-Mikrokapsel und erstem Kopplungselement mit dem in Schritt b) hergestellten Komplex aus antigenpräsentierendem MHC-Klasse-I-Molekül und dem zweiten Kopplungselement unter Bedingungen, die eine Bindung zwischen dem ersten und dem zweiten Kopplungselement erlauben,
d. Inkontaktbringen des in Schritt c) gebildeten Komplexes aus Polyelektrolyt-Mikrokapsel und antigenpräsentierendem MHC-Klasse-I-Molekül mit einer einen T-Zellrezeptor tragenden T-Zelle unter Bedingungen, die eine Bindung zwischen T-Zellrezeptor und antigenpräsentierendem MHC-Klasse-I-Molekül unter Bildung eines Komplexes aus Polyelektrolyt-Mikrokapsel, antigenpräsentierendem MHC-Klasse-I-Molekül und T-Zelle erlauben,
e. Inkontaktbringen des in Schritt d) gebildeten Komplexes aus Polyelektrolyt-Mikrokapsel, antigenpräsentierendem MHC-Klasse-I-Molekül und T-Zelle mit einem fluoreszenzmarkierten Antikörper, der spezifisch an den T-Zell-Rezeptor oder einen Corezeptor des T-Zell-Rezeptors bindet, oder einem fluoreszenzmarkierten Antikörper, der spezifisch an einen zweiten Antikörper bindet, der spezifisch an den T-Zell-Rezeptor oder Corezeptor des T-Zell-Rezeptors bindet, und
f. Messen der Fluoreszenz des Komplexes aus Polyelektrolyt-Mikrokapsel, antigenpräsentierendem MHC-Klasse-I-Molekül und T-Zelle.

Die Herstellung eines gefalteten, ein Antigen präsentierenden MHC-Klasse-I-Moleküls ist beispielsweise in der WO 2013/102458 A1 beschrieben.

Durch gleichzeitige Fluoreszenzmarkierung mindestens einer Komponente des pMHC-I/PEMC-Komplexes und der T-Zelle in dem pMHC-I/PEMC/T-Zell-Komplex kann in vorteilhafter Weise ein Förster-Resonanzenergietransfer (FRET) genutzt werden, um die Detektion zu verbessern. Dazu wird eine Kombination aus mindestens zwei unterschiedlichen Fluoreszenzfarbstoffen eingesetzt, die als Donor- und Akzeptor-Fluoreszenzfarbstoff fungieren können. Die Art und die Abmessungen der genannten Komponenten werden so aufeinander abgestimmt, dass ein Förster-Resonanzenergietransfer (FRET) erfolgen kann. Beispielsweise können die Komponenten so aufeinander abgestimmt werden, dass möglichst geringe Abstände der Donor- und Akzeptor-Fluoreszenzfarbstoffe resultieren.

In einer Ausgestaltung der Erfindung kann der Fluoreszenzfarbstoff im Inneren der Polyelektrolyt-Mikrokapsel beispielsweise ein Donor-Fluoreszenzfarbstoff und der Fluoreszenzfarbstoff, mit dem ein fluoreszenzmarkierter Antikörper markiert ist, ein Akzeptor-Fluoreszenzfarbstoff sein, oder umgekehrt der Fluoreszenzfarbstoff im Inneren der Polyelektrolyt-Mikrokapsel ein Akzeptor-Fluoreszenzfarbstoff und der Fluoreszenzfarbstoff, mit dem der fluoreszenzmarkierte Antikörper markiert ist, ein Donor-Fluoreszenzfarbstoff sein. Vorzugsweise werden ein Donor- und Akzeptor-Fluoreszenzfarbstoff, eine Polyelektrolyt-Mikrokapsel, ein antigenpräsentierendes MHC-Klasse-I-Molekül und ein fluoreszenzmarkierter Antikörper verwendet, so dass in dem Komplex aus Polyelektrolyt-Mikrokapsel, antigenpräsentierendem MHC-Klasse-I-Molekül, T-Zelle und fluoreszenzmarkiertem Antikörper ein Förster-Resonanzenergietransfer ermöglicht wird.

In einem weiteren Aspekt betrifft die vorliegende Erfindung ein Kit zur antigenspezifischen Immunfärbung von T-Zellen. Der Kit in seiner breitesten Auslegung ist im Anspruch 7 beschrieben. Ausführungsformen im Folgenden, bei denen die Polyelektrolyt-Mikrokapsel nicht einen Fluoreszenzfarbstoff eingeschlossen in ihrem Innenraum aufweist, sind lediglich als Referenzbeispiele anzusehen.

Das erfindungsgemäße Kit umfasst mindestens eine Polyelektrolyt-Mikrokapsel mit einem Innenraum und einer den Innenraum umgebenden Polyelektrolyt-Hülle, wobei die Polyelektrolyt-Hülle mindestens einen Peptid/MHC-Klasse-I-Protein-Komplex gebunden aufweist, so dass der Peptid/MHC-Klasse-I-Protein-Komplex mit seiner einen T-Zell-Rezeptor bindenden Bindungsstelle zu dem die Polyelektrolyt-Hülle außen umgebenden Raum gerichtet ist.

Das erfindungsgemäße Kit umfasst mindestens eine Polyelektrolyt-Mikrokapsel, die einen Fluoreszenzfarbstoff aufweist, wobei der Fluoreszenzfarbstoff vorzugsweise in ihren Innenraum eingeschlossen oder an die Polyelektrolyt-Hülle gebunden ist, wobei die Formulierung "an die Polyelektrolyt-Hülle gebunden" auch den Fall umfasst, dass der Fluoreszenzfarbstoff in der Polyelektrolyt-Hülle verankert ist.

In einer besonders bevorzugten Ausführungsform ist bei dem erfindungsgemäßen Kit der mindestens eine Peptid/MHC-Klasse-I-Protein-Komplex indirekt mittels eines Kopplungssystems aus einem ersten Kopplungselement und einem zweiten Kopplungselement an die Polyelektrolyt-Hülle gebunden. Bei dem ersten Kopplungselement handelt es sich bevorzugt um Avidin und/oder Streptavidin, bei dem zweiten Kopplungselement bevorzugt um Biotin.

Das Kit umfasst ferner mindestens einen an eine T-Zelle bindenden fluoreszenzmarkierten primären Antikörper oder einen an eine T-Zelle bindenden nicht fluoreszenzmarkierten primären Antikörper und einen fluoreszenzmarkierten sekundären Antikörper.

Das Kit kann selbstverständlich auch andere Komponenten umfassen, beispielsweise geeignete Pufferlösungen oder Komponenten zu deren Herstellung.

Im Folgenden wird die Erfindung rein zu Veranschaulichungszwecken anhand einer Figur und eines beispielhaften Protokolls für die Durchführung einer Ausführungsform des erfindungsgemäßen Verfahrens näher beschrieben.

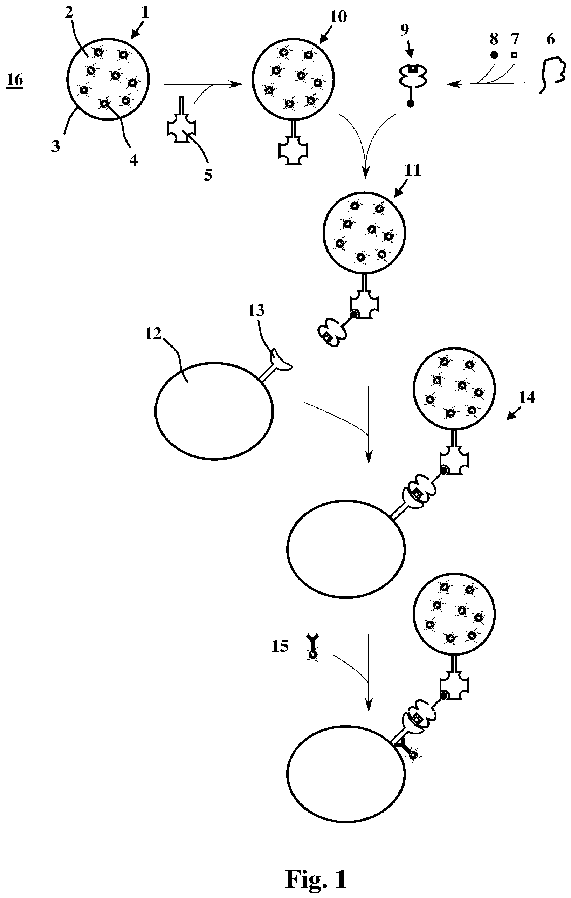

Figur 1 Schematische Darstellung einer Ausführungsform des erfindungsgemäßen Verfahrens.

### I. Beschreibung der Figur

Figur 1 zeigt schematisch eine Ausführungsform des erfindungsgemäßen Verfahrens. Zur besseren Übersicht ist jeweils nur ein Molekül bzw. Komplex etc. dargestellt. Links oben ist eine Polyelektrolyt-Mikrokapsel (PEMC) 1 mit einem Innenraum 2 und einer den Innenraum 2 umgebenden mehrschichtigen Polyelektrolyt-Hülle 3 dargestellt. Die Polyelektrolyt-Mikrokapsel 1 schließt einen Fluoreszenzfarbstoff 4 ein. An die Hülle 4 der PEMC 1 wird nun Streptavidin 5 kovalent gebunden, so dass die Bindungsstellen des Streptavidin 5 zu dem Raum 16 um die PEMC 1 herum weisen und dort zugänglich sind. Der Komplex 10 aus PEMC 1 und Streptavidin 5 wird mit einem Komplex 9 aus einem gefalteten und an Biotin 8 gebundenen MHC-I-Molekül und einem antigenischen Peptid 7 in Kontakt gebracht. Es bildet sich ein PEMC/pMHC-I-Komplex 11. In Figur 1 sind der besseren Übersicht halber lediglich ein Streptavidin- und ein pMHC-I-Molekül dargestellt.

Der Komplex 9 wird gebildet, indem ein ungefaltetes Ausgangs-MHC-I-Molekül 6 gefaltet und ein antigenisches Peptid 7 und Biotin 8 daran gebunden werden. Der Komplex 11 wird mit aus Blut isolierten T-Zellen 12 in Kontakt gebracht, so dass der T-Zell-Rezeptor (TCR) 13 an den pMHC-Komplex 9 binden kann. Der gebildete Komplex 14 aus PEMC 1, pMHC-Molekül 9 und T-Zelle 12 wird mit einem fluoreszenzmarkierten Anti-TCR-Antikörper 15 in Kontakt gebracht. Die Reaktionsmischung wird anschließend einer Durchflusszytometrie unterzogen. Selbstverständlich kann hier auch ein System aus einem primären und einem fluoreszenzmarkierten sekundären Antikörper verwendet werden. Ebenso muss sich der (primäre) Antikörper nicht gegen den TCR richten, sondern kann sich auch gegen einen damit verbundenen Corezeptor, z.B. CD8, richten.

### II. Beispielprotokoll

### 1. Herstellung von Mikrokapseln

### Herstellung der Polymerlösungen

a. Man löse 2 mg/ml PAH (Poly(allylamin-hydrochlorid), MG = 58,000) und PAA (Poly(acrylsäure), MG = 60.000) (0,286 ml 35% w/v) in 0,5 M NaCl-Lösung (in H2O).

### Einschluss des Farbstoffs

a. Man füge 2 ml H₂O zu 6 µM CaCO₃-Partikeln und beschalle für 5 min
b. Man füge 5 µl 1 mg/ml Fluoreszenzfarbstoff (z. B. Dextran oder ein mit einem Fluoreszenzfarbstoff gekoppeltes Protein, beispielsweise Fluorescein oder Tetramethylrhodamin) zu den Partikeln und inkubiere für 30 min unter Schütteln
c. Man schlage die CaCO₃-Partikel für 2 min bei 3000 UPM nieder; Überstand entfernen
d. Man wasche die CaCO₃-Partikel 3x mit H₂O

### Bildung einer mehrschichtigen Hülle:

a. Adsorption von Polymer: 10 min Schütteln bei 1200 UPM (2 ml)
b. Man wasche nach der Adsorption dreimal mit Wasser, füge dann das zweite Polymer hinzu.

### Qualitätskontrolle

a. Man messe die Partikelzahl und Fluoreszenz mittels Durchflusszytometrie (FACS).

### Lagerung

a. Man entferne die EDTA-Lösung durch einen weiteren Zentrifugationsschritt. Die Kapseln können in H₂O bei Raumtemperatur gelagert werden, es sei denn, der eingekapselte Farbstoff benötigt besondere Bedingungen (z.B. Puffer, pH, Temperatur).

### 1. Anheftung von Streptavidin an die Mikrokapseln

Vernetze die Kapseln/Partikel mit 10mg/mL (1-Ethyl-3-[3dimethylaminopropyl]carbodiimid-Hydrochlorid) über Nacht bei RT (Raumtemperatur) in MES (2-(N-Morpholino) ethansulfonsäure)-Puffer, pH 6,0.

Zum Quenching von EDC 3x mit Wasser waschen, bei 3000 UPM für 2 min zentrifugieren.

Aktiviere die -COOH-Gruppe auf den Kapseln mit EDC (0,4 M) und Sulfo-NHS (N-Hydroxysuccinimid, 0,1 M) durch Inkubation für 1 h bei RT (in phosphatgepufferter Salzlösung (PBS), pH-Wert 7,2)

Zum Quenching von EDC 3x mit kaltem Wasser waschen, bei 3000 UPM für 2 min zentrifugieren

Füge Streptavidin zu einer Endkonzentration von 5 µg/mL hinzu und inkubiere unter Schütteln über Nacht bei RT (PBS pH 8,2)

Blockiere die verbleibenden NHS-Ester durch Behandlung mit 50 mM Tris-Cl (pH 8,8) für 30 min bei RT.

Wasche 3x mit Wasser, Zentrifugiere bei 3000 UPM für 2 min.

Qualitätskontrolle: Füge Biotin-Fluorescein und messe die Partikelzahl und Fluoreszenz mittels Durchflusszytometrie (FACS).

### 2. Herstellung von MHC-Klasse I-Proteinen

### Lösungen:

50 ml Saccharoselösung:
   500 µl 1 M Tris-Cl pH 8,0
   12,5 g Saccharose
   100 µl 500 mM EDTA
   Wasser zu 50 ml
   (DTT erst unmittelbar vor Gebrauch zufügen)
50 ml Reinigungspuffer:
   2,5 ml 1 M Tris-Cl pH 8,0
   12,5 g Saccharose
   2,5 ml 20% Triton X-100
   500 µl 500 mM EDTA
   Wasser zu 50 ml
   (DTT erst unmittelbar vor Gebrauch zufügen)
50 ml Harnstoffpuffer:
   20 ml 5 M NaCl (oder: 5,84 g)
   6,01 g Harnstoff
   1,25 ml 1 M Tris-CI pH 8,4
   (DTT erst unmittelbar vor Gebrauch zufügen)
50 ml TBS:
   1,5 ml 5 M NaCl
   1 ml 1 M Tris-Cl pH 7,5
   (füge 1/100 v/v 500 mM PMSF unmittelbar vor Gebrauch hinzu)
   50 ml Harnstoff-Resolubilisierungspuffer
   24 g Harnstoff
   2,5 ml 1 M HEPES pH 6,5

### Verfahren

Ziehe 20-ml-Kulturen E. coli, Stamm Rosetta oder BL21, enthaltend die schwere Kette, β2m oder das H-2Db/hβ2m-Einzelketten-Konstrukt in einem pGMT7 Vektor, in LB-Medium über Nacht bei 37 °C, unter Selektion durch 100 µg/ml Ampicillin und Chloramphenicol, heran. Beispiele: β2m-Expressionsplasmid pHN1+, in SPB542 (Expressionsstamm, abgeleitet von BL21); H-2Db-Volllängen-Expressionsplasmid pGMT7Db, in SPB595 (Rosetta). Siehe Sunil Kumar Saini, Esam Tolba Abualrous, Anca-Sarmiza Tigan, Kathryn Covella, Ursula Wellbrock, Sebastian Springer (2013), Not all empty MHC class I molecules are molten globules: Tryptophan fluorescence reveals a two-step mechanism of thermal denaturation, Molecular Immunology, 54, 386-396, http://dx.doi.org/10.1016/j.molimm.2013.01.004.

Zentrifugiere bei 3000 x g für 10 Minuten, und verwende den Niederschlag, um 1 L-Kulturen der gleichen Zusammensetzung anzuimpfen.

Ziehe die 1-L-Kulturen zu einer OD600 zwischen 0,4 und 0,5 an, füge dann IPTG (Isopropyl-β-D-1-thiogalactopyranosid) zu, um die Expression zu induzieren (Endkonzentration 0,5 mM).

Ziehe die Kulturen über 4 Stunden zu einer OD600 von 1,0 an, bevor durch Zentrifugation bei 13.000 × g für 10 Minuten geerntet wird

Die folgenden Mengen sind für ein einzelnes Pellet aus einer 1-Liter-Kultur.

Resuspendiere das Pellet in 20 ml Saccharoselösung (25% Saccharose, 1 mM EDTA, 1 mM PMSF und 10 mM DTT, 10 mM Tris, pH 8,0). Überführe in 50 ml Falcon-Röhrchen und friere zur Aufbewahrung bei -20 ° C ein.

Lysiere die Zellen durch Auftauen in einem 30-°C- Wasserbad. Schwenke die Röhrchen, um sicherzustellen, dass das Auftauen schnell und gleichmäßig erfolgt. Halte die Proben von nun an immer bei 4 °C (auf Eis).

Beschalle (kleine Spitze, 3 x 20 Sekunden), bis die Lösung nicht mehr viskos ist. Nehme eine 50 µl Probe aus dem Lysat und beschrifte sie mit #1.

Zentrifugiere die Zelllysate in Oak-Ridge-Röhrchen (JA25.50 Rotor) bei 40.000 x g, 4 °C für 15 Minuten. Nehme eine Probe aus dem Überstand und beschrifte sie #2. Überstand verwerfen.

Resuspendiere das Pellet in 20 ml Detergenspuffer (25% Saccharose, 1% Triton X100, 5 mM EDTA und 2 mM DTT, 50 mM Tris, pH 8,0) durch Ultraschallbehandlung wie oben. Nehme eine Probe aus der Suspension und beschrifte sie mit #3. Zentrifugiere wie oben, und verwerfe den Überstand.

Wiederhole diesen Vorgang der Resuspendierung, Ultraschallbehandlung und Zentrifugation für zwei weitere Waschschritte in
a. Harnstoff-Puffer (2 M NaCl, 2 M Harnstoff und 2 mM DTT, 25 mM Tris, pH 8,4) (Probe #4) und
b. Tris-gepufferter Kochsalzlösung (150 mM NaCl und 0,5 mM PMSF, 20 mM Tris, pH 7,5) (Probe #5).

Nach dem letzten Waschschritt resuspendiere das Pellet in 10 ml 8 M Harnstoff (enthaltend 50 mM HEPES pH 6,5 und 100 µM β-Mercaptoethanol) durch Ultraschallbehandlung (Probe #6), und löse durch sanftes Schütteln bei 4 ° C.

Nach 48 Stunden zentrifugiere die gelöste Proteinsuspension bei 40.000 g, 4 °C, für 15 Minuten. Gegebenenfalls muss ein zweites Mal zentrifugiert oder sogar durch ein 0,22-µm-Filter filtriert werden, um das unlösliche Sediment loszuwerden.

Aliquotiere den Überstand (Probe #7) und lagere bei minus 20 °C

Um die Qualität des Verfahrens zu beurteilen, lasse jeweils 1 µl der Proben #1-7 auf einem SDS-PAGE-Gel mit einem Größenmarker und etwas gereinigtem Protein aus einer früheren Aufbereitung laufen

Bestimme die Konzentration des gereinigten Proteins durch Extinktion:
a. Verdünne 1/20 in 6 M Guanidinium-Hydrochlorid.
b. Messe die Extinktion bei 280 nm gegen Wasser.
c. Berechne den Extinktionskoeffizienten aus der Sequenz des Proteins mit einem Online-Werkzeug wie http://www.basic.northwestern.edu/biotools/proteincalc.html. (Beispielsweise kann der Extinktionskoeffizient der lumenalen Domäne der schweren Kette von D^{b} 84830 M⁻¹cm⁻¹ betragen (und das Molekulargewicht beträgt 33025 g/mol, so dass eine 1 mg/ml Lösung eine E280 von 2,57 hat).
d. Anhand des berechneten Extinktionskoeffizienten und der eigenen Messung wird die Konzentration und die Ausbeute des Proteins ermittelt. Die Ausbeute aus einem Liter Kultur sollte in einem Bereich von 15-25 mg Protein liegen.

### D. Faltung und Biotinylierung von MHC-Klasse-I-Proteinen

### 1. Materialien:

HLA-Schwerkette-Einschlusskörper, 20 oder mehr mg/ml in Harnstoffpuffer, gelagert bei -80 °C, 8-mg-Aliquots
MHC-Leichtkette-Einschlusskörper, 10 oder mehr mg/ml in Harnstoffpuffer, gelagert bei -80 °C, 7-mg-Aliquots,
Untersuchungspeptid: 2,5-7,5 mg
Rückfaltungspuffer: Mag gebe in einen 250 ml-Kolben (Schott, blauer Deckel) mit flachem Boden: 17,42 g L-Arginin-Base (Fluka), 5,95 g HEPES (Roth), 1 ml EDTA (Sigma) 0,5 M pH 8, 200 ml dd H₂O, Magnetrührstab. Lege bis zum Erreichen von 10 °C auf Eis, stelle dann den pH-Wert mit konzentrierter HCl (in der Regel 5 bis 10 ml) auf pH 7,76, fülle mit dd H₂O auf 250 ml auf, autoklaviere, lagere bei 4 °C
TBS: 20 mM Tris (Sigma), pH 8,0, 4 °C, 150 mM NaCl (Merck). Filtrieren und Autoklavieren, Lagerung bei 4 °C.
Harnstoffpuffer: 8 M Harnstoff (UCB), 10 mM Tris, pH 8 (Sigma), 100 mM NaH₂PO₄ (Merck), 0,1 mM EDTA (Roth), 0,1 mM DTT. Lagere Harnstoff als 10 M Stammlösung mit "Duolite"-Indikatorharz (Merck) und filtriere unmittelbar vor der Verwendung.
Injektionspuffer: 3 M Guanidin-HCl (Fluka), 10 mM Na-Acetat (Sigma), 10 mM EDTA (Sigma), mit HCl auf pH 4,2 einstellen und als 10-ml-Aliquots bei -80 ° C lagern
Regenerationspuffer für Amicon-Membran: 0,1 M NaOH, 100 ppm NaOCl Reduziertes Glutathion (Sigma)
Oxidiertes Glutathion (Sigma) 1000 x PMSF (Sigma): 200 mM Stammlösung in Methanol (Merck), lagere 250-µl-Aliquots bei -80 °C
1000 x Leupeptin (Roche): 1 mg/ml Stammlösung in H₂O, lagere 250-µl-Aliquots bei -80 °C
1000 x Pepstatin (Roche): 0,7 mg/ml Stammlösung in Methanol (Merck), lagere 250-µl-Aliquots bei -80 °C
BirA-Enzym (Affinity Biosciences, oder selbst hergestellt). Nach dem Auftauen nicht wieder einfrieren; Lagerung bei -80 ° C
D-Biotin (Sigma) 100 mM in 200 mM Tris (Sigma) 35-µl-Aliquots, Lagerung bei -80 °C
ATP 100 mM in H₂O auf pH 7,0 eingestellt, Lagerung von 300-µl-Aliquots bei -80 °C.
MgCl₂ 1 M
Tris 1 M
Protein-Assay-Kit (z.B. Roti-Nanoquant).
1 Millipore PBTK-Membran, 76 mm Durchmesser, NMWL 30.000
3 Millipore Amicon Ultra-15-Aufkonzentrierungsvorrichtungen (MWCO = 10 kDa-Membran)
1 Millipore Stericup Express 0,22 µm Vakuumfiltrationsvorrichtung (250 ml) .
FPLC-System mit Superdex-75-Säule (26 mm Durchmesser), Fraktionssammler und UV-Recorder, (z.B. Pharmacia)

### 2. Verfahren: (alle Schritte auf Eis oder bei 4 ° C wenn nicht anders angegeben)

### Tag 1 (Rückfaltung Start)

- Wiege 2,5 bis 7,5 mg Peptid ab, füge DMSO zu 10 mg/ml hinzu. Träufle rasch mit 1-ml-Pipette, damit es nicht einfriert.
- Füge Folgendes zu einer Flasche Rückfaltungspuffer:
   385 mg reduziertes Glutathion
   77,5 mg oxidiertes Glutathion
   250 µl PMSF 1000x (vorsichtig öffnen: Methanol)

### Peptid-Lösung

- Taue 8 mg MHC-Schwerkette auf und füge 8 M Harnstoff zu 20 mg/ml hinzu. Füge 700 µl Injektionspuffer hinzu. Lade in eine 1-ml-Spritze mit einer 26G-Nadel. Rühre den Rückfaltungspuffer mit hoher Geschwindigkeit und spritze die schwere Kette kräftig und so nahe am Rührer wie möglich hinein.
- Taue 7 mg MHC-Leichtkette auf und füge 780 µl Injektionspuffer hinzu. Behandle wie die schwere Kette.
- Inkubiere die Reaktionsmischung über Nacht (12 h im Idealfall) bei 10 °C unter leichtem Schütteln oder Rühren

### Tag 2 (Rückfaltung)

- Füge 8 mg schwere Kette (keine leichte Kette!) Mit Harnstoff- und Injektionspuffer wie oben beschrieben einmal am Morgen und einmal am Abend (12-h-Intervalle im Idealfall)

### Tag 3 (Konzentration, und Beginn der zweiten Rückfaltung)

Die Reaktionslösung wird durch ein 0,22-µm-Vakuum-Filter (Flaschenaufsatzfilter) in einen neuen 250-ml-Kolben filtriert. Eine Amicon-Rührzelle wird zusammengebaut und mit etwa 200 ml filtriertem dd H₂O bei 60 psi gespült. Die Rückfaltungsreaktionsmischung wird bei 60 psi in der Amicon-Rührzelle auf etwa 20 ml eingeengt. Das Retentat wird in einem 50-ml-Falcon-Röhrchen gesammelt und die Rührzelle mit Permeat gespült, bis etwa 25 ml Retentat vorliegt. Die Amicon-Zelle wird zerlegt und mit 70% EtOH gereinigt.

Das Permeat wird für die zweite Rückfaltungsreaktion gesammelt. Diese wird durch Zugabe von 250 µl l PMSF 1000x zum Permeat erreicht. Zu 8 mg aufgetauter MHC-Schwerkette wird 8 M Harnstoff zu 20 mg/ml gegeben, und mit 700 µl Injektionspuffer gemischt. Die Mischung wird dann in eine 1-ml-Spritze mit einer 26G-Nadel aufgenommen und zum Permeat hinzugegeben. Schließlich wird 7 mg aufgetaute MHC-Leichtkette mit 780 µl Injektionspuffer gemischt und in der gleichen Weise wie die schwere Kette zum Permeat gegeben. Das Permeat wird während der Zugabe der schweren und leichten Kette bei hoher Geschwindigkeit gerührt. Die Reaktionsmischung wird dann für 12 h bei 10 °C unter sanftem Schütteln inkubiert.

### Tag 4 (Rückfaltung)

- Man füge 8 mg schwere Kette (keine leichte Kette!) mit Harnstoff- und Injektionspuffer wie oben beschrieben einmal am Morgen und einmal am Abend (12-h-Intervalle im Idealfall) hinzu.

### Tag 5 (Konzentration)

- Filtriere die Reaktionsmischung aus der zweiten Rückfaltung durch 0,22-µm-Vakuumfilter (von Tag 3) (Flaschenaufsatzfilter) in einen neuen 250-ml-Kolben. Entsorge den Filter.
- Montiere die Amicon-Rührzelle und spüle mit rund 200 ml filtriertem dd H₂0 bei 60 psi.
- Konzentriere die filtrierte Reaktionsmischung auf 20 ml bei 60 psi in der gerührten Amicon-Rührzelle. Sammle das Retentat und spüle die Rührzelle mit Permeat, bis 25 ml Retentat vorliegen.
- Zerlege die Amicon-Zelle und reinige alle Teile mit 70% EtOH
- Zentrifugiere die Retentate (von Tag 3 und 5) bei 3200 g für 5 Minuten
- Konzentriere die zentrifugierten Retentate der Rührzelle schrittweise in der Amicon-Ultra-15-Vorrichtung, bis 4-5 ml Retentat vorliegen
- Spüle die Vorrichtung mit ca. 1 ml Permeat, bis insgesamt 5-6 ml Retentat vorliegen.

### Tag 6 (FPLC, Konzentration, Biotinylierung)

- Zentrifugiere das Retentat von Tag 5 bei 3200 g für 5 Minuten
- Führe mit dem 5-6 ml Retentat eine Größenausschluss-Chromatographie auf einer S75-Säule (TBS als Puffer) durch. Sammle 4-ml-Fraktionen aus 100-200 ml. Zeichne die UV-Absorption bei 280 nm auf.
- Sammle Fraktionen des Monomer-Peaks in einem 50-ml-Falcon-Röhrchen und gebe sofort PMSF, Leupeptin und Pepstatin zu l x Endkonzentration hinzu
- Konzentriere in einem Ultra-15-Gerät auf 3-4 ml. Spüle mit Permeat, bis 5,0 ml Retentat vorliegen.
- Hinzufügen: 400 µl 1 M Tris pH 8 25 °C, 25 µl 1 M MgCl₂, 250 µl ATP 100 mM. Mische durch Umdrehen des Röhrchens.
- Füge 10-20 µg BirA Enzym und 28,5 µl 100 mM Biotin hinzu. Gut mischen durch mehrmaliges Umdrehen des Röhrchens.
- Inkubation für 12-16 Stunden in einem Wasserbad bei 27,0 °C

### Tag 7 (FPLC, Konzentration, Protein-Quantifizierung und Lagerung)

- Zentrifugiere die Biotinylierungsreaktionsmischung bei 3200 g für 5 Minuten
- Führe eine weitere FPLC-Trennung mit der Biotinylierungsreaktionsmischung auf einer S75-Säule (TBS als Puffer) durch, und sammle Fraktionen und zeichne die UV-Werte auf, wie oben beschrieben
- Sammle biotinylierten Monomerpeak und füge sofort PMSF, Leupeptin und Pepstatin zu lx Endkonzentration hinzu
- Prääquilibriere mit der Ultra-15-Vorrichtung mit 5 ml TBS. Konzentriere den Peak auf 250-350 µl. Sammle das Retentat und spüle das Gerät mit 100 µl Permeat
- Bestimme die Proteinkonzentration unter Verwendung eines handelsüblichen Standard-Proteinbestimmungskits.
- Stelle die Proben mit TBS auf 2,0 mg/ml Endkonzentration an
- Aliquotiere zu 50-µg-Aliquots in ordnungsgemäß gekennzeichneten lichtundurchlässigen Röhrchen. Lagere bei -80 °C

### E. Anheftung der biotinylierten MHC-Klasse-I-Proteine an Mikrokapseln

1. Füge biotinylierte MHC-Klasse-I-Proteine hinzu und Inkubation für 2 Stunden
2. Wasche 3 x mit Wasser, Zentrifugiere bei 3000 UPM für 2 min
3. Löse die Partikel mit 0,2 M EDTA (pH 7,2) für 30 Minuten auf
4. Qualitätskontrolle I: Füge Antikörper gegen Klasse I und fluoreszenzmarkierten sekundären Antikörper hinzu und messe Partikelzahl und Fluoreszenz mittels Durchflusszytometrie (FACS).
5. Qualitätskontrolle II: Tausche das an Klasse-I-Protein gebundene Peptid gegen ein Peptid, das mit einem Fluorophor wie Fluorescein oder Tetramethylrhodamin markiert ist, und messe Partikelzahl und Fluoreszenz mittels Durchflusszytometrie (FACS)

### F. Isolierung von Lymphocyten aus Blut

### Material:

Schritt 1-3: 60 ml Ficoll, ca.150 ml PBS (-Ca-Mg)
Schritt 5: Einfriermedium; Kryoröhrchen

### Schritt 1:

- 4x 15 ml Ficoll pro Falcon-Röhrchen vorlegen
- Blut in 150 ml-Zellkulturflasche einfüllen
- Blut mit PBS auf 125 ml auffüllen
- Je 30 ml Blutgemisch über Ficoll in ein Falcon-Röhrchen schichten
- Zentrifugieren: 2000 UPM, 30 min, 21°C (keine Bremse).

### Schritt 2:

- Thrombozyten/PBS-Schicht bis auf 5ml von oben abnehmen
- Lymphozytenschicht abnehmen (höchstens 10 ml), Fraktionen von 2 Falcon-Röhrchen in einem sammeln; mit PBS auf 50ml auffüllen
- Zentrifugieren: 1500 UPM, 10 min, 20 °C

### Schritt 3:

- Waschschritt: auf 50 ml mit PBS auffüllen, 1300 UPM, 10 min zentrifugieren.

### Schritt 4:

- Lymphozyten in einem Falcon-Röhrchen sammeln, auf 50 ml auffüllen
- Probe zum Zellen zählen entnehmen
- Zentrifugieren: 1100 UPM, 10 min, 20 °C

### Schritt 5:

- Zellzahl bestimmen:
   10 µl Zellen mit 90 µl PBS versetzen.
   10 µl dieser Zellsuspension mit 10 µl einer 0.4%-Lösung von Trypanblau (CAS 72-57-1) in PBS mischen.
   Mischung in ein Haemocytometer nach Neubauer pipettieren und Zellen zählen.

### Schritt 6:

- Einfrieren, beschriften: möglichst viele 10⁸/2ml-Zelleinheiten. Die letzte 10⁸/2ml-Einheit wird mit 3 ml verdünnt und in Form von 5 x 2^{∗}10⁷/1ml eingefroren

### G. Färbung der Lymphocyten mit Microkapseln

### Materialien:

PFEA ("FACS-Puffer"): 500 ml PBS; 2% FeS; 2 mM EDTA; 0,02% NaN₃ PBS-EDTA: 500 ml PBS; 2 mM EDTA; 0,02% NaN₃
Tetramer-Färbungspuffer (TSB), auch Tetramer-Lösung genannt: 50% PBS; 50% FeS; 2 mM EDTA

### Färbung:

### 1. Waschschritt:

- In 96-Napf-Platte etwa 0.5-l x 10⁶ Zellen ausplattieren und abzentrifugieren (4 °C, 2 min, 1800 UPM). Anschließend Platte abflicken.

### 2. Lebend-/Tot-Färbung (optional)

- Waschschritt mit PBS-EDTA (Zellen in 150 µl aufnehmen und abzentrifugieren)
- Aqua live/dead 1:400 oder 1:200 (wurde Aqua live/dead vor <2 Wochen eingefroren 1:400, bei 2-4 Wochen 1:200) in PBS-EDTA verdünnen.
- 50 µl pro Napf resuspendieren
- Inkubation 20 min bei 4 °C im Dunkeln
- Waschschritt mit PBS-EDTA

### 3. Tetramer-Färbung

- Pro Napf: 10⁵-10⁸ MHC-Mikrokapseln (Optimierung je nach Target) in TSB ansetzen und mischen.
- Pro Napf werden die Zellen in 50 µl der angesetzten Lösung resuspendiert (Lösung vorsichtig aus dem Überstand abnehmen! Pellett nicht aufwirbeln)
- Inkubation 30 min bei RT im Dunklen
- Ein Waschschritt mit PFEA

### 4. Oberflächenmoleküle färben

- Antikörper (z.B. CDB-PerCP) in PFEA ansetzen
- Färbung in 50 µl/well resuspendieren
- Inkubation 20 min bei 4 °C im Dunklen
- Ein Waschschritt mit PFEA
- Zellen in 200 µl PFEA aufnehmen, falls am gleichen Tag gemessen wird. Ansonsten fixieren

### 5. Fixieren:

- Zellen in 100 µl PFEA mit 1% Formaldehyd resuspendieren
- Inkubation 20 min bei 4 °C im Dunklen
- Ein Waschschritt mit PFEA
- Zellen in 200 µl PFEA aufnehmen, bei 4°C im Dunkeln lagern

### 6. Durchflusszytometrie

- Für jede Probe 200 000 Zellen verwenden.
- Nach dem Mischen von Zellen und Tetrameren werden die Proben für 30 Minuten bei 37 °C inkubiert.
- Danach Zellen auf ein Volumen von 1,5 ml mit PBS-Natriumazid auffüllen, in ein entsprechendes Probenröhrchen überführen und in der Durchflusszytometrie analysieren.
- Hierzu werden beispielsweise das Gerät CyFlowSpace von Partec und die Software FloMax Version 2.82 verwendet.
- Die Achseneinstellung der verschiedenen Graphen kann beispielsweise wie folgt erfolgen:
   - - Histogramm 1: Counts / forward scatter (FSC)
   - - Histogramm 2: Counts / side scatter (SSC)
   - - Histogramm 3: SSC / FSC
   - - Histogramm 4: Counts / Fluoreszenz 1 (FL1: 516-556 nm; logarithmische Skala)
   - - Histogramm 5: Counts / Fluoreszenz 3 (FL3: 650-700 nm; logarithmische Skala)
   - - Histogramm 6: FL1 / FSC
   - - Histogramm 7: FL3 / FL1 (logarithmische Skala)
   - - Histogramm 8: FL3 / FSC
- Zunächst werden unbehandelte Zellen zur Bestimmung der Größe der Zellen und der Hintergrundfluoreszenz gemessen. Dazu wird im Histogramm 3 die Region mit den intakten Zellen mit dem "Gate R1" markiert. Diese Region wird auf alle anderen Zellzählungen Histogramme angewendet. In Folge werden dann nur noch Zellen aus der markierten Region in den Histogrammen gezeigt.
- Im nächsten Schritt legt man ein im Histogramm 4 den Schwellenwert RN1 fest, über dem die Zellen als fuoreszenzmarkiert gelten.
- Die Proben werden dann mit diesen Einstellungen gemessen.

## Patentansprüche

1. Verfahren zur antigenspezifischen Immunfärbung von T-Zellen, wobei
- eine Polyelektrolyt-Mikrokapsel, die außen ein antigenpräsentierendes MHC-Klasse-I-Molekül und innen einen ersten Fluoreszenzfarbstoff aufweist, mit einer einen T-Zell-Rezeptor tragenden T-Zelle unter Bedingungen in Kontakt gebracht wird, die eine Wechselwirkung zwischen dem T-Zell-Rezeptor und dem antigenpräsentierenden MHC-Klasse-I-Molekül erlauben, so dass ein Komplex aus T-Zelle, antigenpräsentierendem MHC-Klasse-I-Molekül und Polyelektrolyt-Mikrokapsel gebildet werden kann,
- ein mit einem zweiten Fluoreszenzfarbstoff markierter Antikörper direkt oder indirekt an die T-Zelle in dem Komplex aus T-Zelle, antigenpräsentierendem MHC-Klasse-I-Molekül und Polyelektrolyt-Mikrokapsel gebunden wird,
- der erste und zweite Fluoreszenzfarbstoff unterschiedlich sind und als Donor- und Akzeptor-Fluoreszenzfarbstoff fungieren können, und
- ein Donor- und Akzeptor-Fluoreszenzfarbstoff, eine Polyelektrolyt-Mikrokapsel, ein antigenpräsentierendes MHC-Klasse-I-Molekül und ein mit einem zweiten Fluoreszenzfarbstoff markierter Antikörper verwendet werden, so dass in dem Komplex aus Polyelektrolyt-Mikrokapsel, antigenpräsentierendem MHC-Klasse-I-Molekül, T-Zelle und mit einem zweiten Fluoreszenzfarbstoff markierter Antikörper ein Förster-Resonanzenergietransfer ermöglicht wird.

2. Verfahren nach Anspruch 1, wobei das antigenpräsentierende MHC-Klasse-I-Molekül direkt oder indirekt mittels kovalenter und/oder nicht-kovalenter Bindung an die Polyelektrolyt-Mikrokapsel gebunden ist

3. Verfahren nach Anspruch 2, wobei Avidin und/oder Streptavidin direkt an die Polyelektrolyt-Mikrokapsel und Biotin direkt an das antigenpräsentierende MHC-Klasse-I-Molekül gebunden ist

4. Verfahren nach einem der vorhergehenden Ansprüche, wobei
a) der Fluoreszenzfarbstoff im Inneren der Polyelektrolyt-Mikrokapsel ein Donor-Fluoreszenzfarbstoff und der zweite Fluoreszenzfarbstoff, mit dem der Antikörper markiert ist, ein Akzeptor-Fluoreszenzfarbstoff ist, oder b) der Fluoreszenzfarbstoff im Inneren der Polyelektrolyt-Mikrokapsel ein Akzeptor-Fluoreszenzfarbstoff und der zweite Fluoreszenzfarbstoff, mit dem der Antikörper markiert ist, ein Donor-Fluoreszenzfarbstoff ist.

5. Verfahren nach Anspruch 4, wobei ein mit einem zweiten Fluoreszenzfarbstoff markierter Antikörper verwendet wird, der spezifisch an den T-Zell-Rezeptor oder einen Corezeptor des T-Zell-Rezeptors bindet, oder ein mit einem zweiten Fluoreszenzfarbstoff markierter Antikörper verwendet wird, der spezifisch an einen zweiten Antikörper bindet, der spezifisch an den T-Zell-Rezeptor oder Corezeptor des T-Zell-Rezeptors bindet.

6. Verfahren nach einem der Ansprüche 4 bis 5, umfassend das Messen der Fluoreszenz des Komplexes aus Polyelektrolyt-Mikrokapsel, antigenpräsentierendem MHC-Klasse-I-Molekül, T-Zelle und mit einem zweiten Fluoreszenzfarbstoff markierten Antikörper.

7. Kit zur antigenspezifischen Immunfärbung von T-Zellen, umfassend
a) mindestens eine Polyelektrolyt-Mikrokapsel (1) mit einem Innenraum (2) und einer den Innenraum (2) umgebenden Polyelektrolyt-Hülle (3), wobei die Polyelektrolyt-Hülle (3) mindestens einen Peptid/MHC-Klasse-I-Protein-Komplex gebunden aufweist, so dass der Peptid/MHC-Klasse-I-Protein-Komplex mit seiner einen T-Zell-Rezeptor (13) bindenden Bindungsstelle zu dem die Polyelektrolyt-Hülle (3) außen umgebenden Raum (16) gerichtet ist, und wobei die Polyelektrolyt-Mikrokapsel (1) einen Fluoreszenzfarbstoff (4) eingeschlossen in ihrem Innenraum (2) aufweist, und
b) mindestens einen mit einem zweiten Fluoreszenzfarbstoff markierten primären Antikörper (15) oder einen nicht mit einem zweiten Fluoreszenzfarbstoff markierten primären Antikörper und einen mit einem zweiten Fluoreszenzfarbstoff markierten sekundären Antikörper.

8. Kit nach Anspruch 9, wobei der mindestens eine Peptid/MHC-Klasse-I-Protein-Komplex indirekt mittels eines Kopplungssystems aus einem ersten Kopplungselement (5) und einem zweiten Kopplungselement (8) an die Polyelektrolyt-Hülle (3) gebunden ist, wobei das erste Kopplungselement (5) bevorzugt Avidin und/oder Streptavidin, und das zweite Kopplungselement (8) bevorzugt Biotin ist.

## Claims

1. A method for antigen-specific immunostaining of T-cells, wherein
- a polyelectrolyte micro-capsule, which comprises an antigen-presenting MHC class I molecule on the outside and a first fluorescent dye on the inside, is brought into contact with a T-cell carrying a T-cell receptor under conditions that allow an interaction between the T-cell receptor and the antigen-presenting MHC class I molecule, so that a complex consisting of the T-cell, the antigen-presenting MHC class I molecule and the polyelectrolyte micro-capsule can be formed,
- an antibody marked with a second fluorescent dye is bound directly or indirectly to the T-cell in the complex consisting of the T-cell, the antigen-presenting MHC class I molecule and the polyelectrolyte micro-capsule,
- the first and the second fluorescent dye are different and can serve as donor and acceptor fluorescent dyes, and
- a donor and acceptor fluorescent dye, a polyelectrolyte micro-capsule, an antigen-presenting MHC class I molecule and an antibody marked with a second fluorescent dye are used, so that a Forster resonance energy transfer is made possible in the complex consisting of the polyelectrolyte micro-capsule, the antigen-presenting MHC class I molecule, the T-cell and the antibody marked with a second fluorescent dye.

2. The method according to claim 1, wherein the antigen-presenting MHC class I molecule is directly or indirectly bound to the polyelectrolyte micro-capsule by means of a covalent and/or non-covalent bond.

3. The method according to claim 2, wherein avidin and/or streptavidin is directly bound to the polyelectrolyte micro-capsule and biotin is directly bound to the antigen-presenting MCH class I molecule.

4. The method according to one of the afore-mentioned claims, wherein
a) the fluorescent dye inside the polyelectrolyte micro-capsule is a donor fluorescent dye and the second fluorescent dye with which the antibody is marked is an acceptor fluorescent dye, or b) the fluorescent dye inside the polyelectrolyte micro-capsule is an acceptor fluorescent dye and the second fluorescent dye with which the antibody is marked is a donor fluorescent dye.

5. The method according to claim 4, wherein an antibody marked with a second fluorescent dye is used, which binds specifically to the T-cell receptor or a coreceptor of the T-cell receptor, or an antibody marked with a second fluorescent dye is used, which binds specifically to a second antibody, which binds specifically to the T-cell receptor or coreceptor of the T-cell receptor.

6. The method according to one of the claims 4 to 5, which comprises measuring the fluorescence of the complex consisting of the polyelectrolyte micro-capsule, the antigen-presenting MHC class I molecule, the T-cell and the antibody marked with a second fluorescent dye.

7. A kit for antigen-specific immunostaining of T-cells comprising
a) at least one polyelectrolyte micro-capsule (1) with an interior space (2) and a polyelectrolyte shell (3) surrounding the interior space (2), wherein the polyelectrolyte shell (3) comprises at least one bound peptide/MHC class I protein complex, so that the peptide/MHC class I protein complex is oriented with its binding site binding a T-cell receptor (13) towards the space (16) surrounding the polyelectrolyte shell (3) on the outside, and wherein the polyelectrolyte micro-capsule (1) comprises a fluorescent dye (4) enclosed in its interior space (2), and
b) at least one primary antibody (15) marked with a second fluorescent dye or a primary antibody not marked with a second fluorescent dye and a secondary antibody marked with a second fluorescent dye.

8. The kit according to claim 7, wherein the at least one peptide/MHC class I protein complex is indirectly bound with the polyelectrolyte shell (3) by means of a coupling system consisting of a first coupling element (5) and a second coupling element (8), wherein the first coupling element (5) is preferably avidin and/or streptavidin, and the second coupling element (8) is preferably biotin.

## Revendications

1. Procédé de coloration immunologique de lymphocytes T spécifiques à un antigène, où
- une microcapsule polyélectrolytique qui comporte à l'extérieur une molécule CMH de classe I présentant l'antigène et à l'intérieur un premier colorant fluorescent est mise en contact avec un lymphocyte T portant un récepteur de lymphocyte T dans des conditions permettant une interaction entre le récepteur de lymphocyte T et la molécule CMH de classe I présentant l'antigène, de manière à pouvoir former un complexe constitué par un lymphocyte T, une molécule CMH de classe I présentant l'antigène et une microcapsule polyélectrolytique,
- un anticorps marqué avec un second colorant fluorescent est lié directement ou indirectement au lymphocyte T dans le complexe constitué par le lymphocyte T, la molécule CMH de classe I présentant l'antigène et la microcapsule polyélectrolytique,
- le premier et le second colorant fluorescent sont différents et peuvent servir de colorant fluorescent donneur et accepteur, et
- un colorant fluorescent donneur et accepteur, une microcapsule polyélectrolytique, une molécule CMH de classe I présentant l'antigène et un anticorps marqué avec un second colorant fluorescent sont utilisés de manière à permettre un transfert d'énergie par résonance de type Förster dans le complexe constitué par la microcapsule polyélectrolytique, la molécule CMH de classe I présentant l'antigène, le lymphocyte T et avec un anticorps marqué avec un second colorant fluorescent.

2. Procédé selon la revendication 1, où la molécule CMH de classe I présentant l'antigène est liée directement ou indirectement au moyen d'une liaison covalente et/ou d'une liaison non-covalente à la microcapsule polyélectrolytique.

3. Procédé selon la revendication 2, où de l'avidine et/ou de la streptavidine sont liées directement à la microcapsule polyélectrolytique et de la biotine est liée directement à la molécule CMH de classe I présentant l'antigène.

4. Procédé selon l'une des revendications précédentes, où
a) le colorant fluorescent à l'intérieur de la microcapsule polyélectrolytique est un colorant fluorescent donneur et le second colorant fluorescent, avec lequel l'anticorps est marqué, est un colorant fluorescent accepteur, ou b) le colorant fluorescent à l'intérieur de la microcapsule polyélectrolytique est un colorant fluorescent accepteur et le second colorant fluorescent, avec lequel l'anticorps est marqué, est un colorant fluorescent donneur.

5. Procédé selon la revendication 4, dans lequel est utilisé un anticorps marqué avec un second colorant fluorescent qui se lie spécifiquement au récepteur de lymphocyte T ou à un co-récepteur du récepteur de lymphocyte T, ou bien un anticorps marqué avec un second colorant fluorescent qui se lie spécifiquement à un second anticorps qui se lie spécifiquement au récepteur de lymphocyte T ou au co-récepteur du récepteur de lymphocyte T.

6. Procédé selon la revendication 4 à 5, comprenant la mesure de la fluorescence du complexe constitué par la microcapsule polyélectrolytique, la molécule CMH de classe I présentant l'antigène, le lymphocyte T et l'anticorps marqué avec un second colorant fluorescent.

7. Kit de coloration immunologique de lymphocytes T, comprenant
a) au moins une microcapsule polyélectrolytique (1) avec un espace intérieur (2) et une enveloppe polyéletrolytique (3) entourant l'espace intérieur (2), où l'enveloppe polyélectrolytique (3) comporte au moins un complexe peptide/protéine CMH de classe I lié, de telle manière que le complexe peptide/protéine CMH de classe I est orienté avec son site de liaison liant un récepteur de lymphocyte T (13) vers l'espace (16) entourant l'enveloppe polyélectrolytique (3) à l'extérieur, et où la microcapsule polyélectrolytique (1) comporte un colorant fluorescent (4) enfermé dans son espace intérieur (2), et
b) au moins un anticorps (15) primaire marqué avec un second colorant fluorescent ou un anticorps primaire non marqué avec un second colorant fluorescent et un anticorps secondaire marqué avec un second colorant fluorescent.

8. Kit selon la revendication 9, où l'au moins un complexe peptide/protéine CMH de classe I est indirectement lié à l'enveloppe polyélectrolytique (3) au moyen d'un système de couplage constitué par un premier élément de couplage (5) et un second élément de couplage (8), où le premier élément de couplage (5) est de préférence de l'avidine et/ou de la streptavidine, et le second élément de couplage (8) est de préférence de la biotine.
